## Europäisches Patentamt
## European Patent Office
## Office européen des brevets

(11) Veröffentlichungsnummer: **0 079 046**
**A1**

(12) # EUROPÄISCHE PATENTANMELDUNG

(21) Anmeldenummer: 82110148.2

(22) Anmeldetag: 04.11.82

(51) Int. Cl.³: **A 01 G 9/02**
**A 01 G 7/00**

(30) Priorität: 07.11.81 DE 8132649 U

(43) Veröffentlichungstag der Anmeldung:
18.05.83 Patentblatt 83/20

(84) Benannte Vertragsstaaten:
AT BE CH DE FR GB IT LI LU NL SE

(71) Anmelder: Biotest-Serum-Institut GmbH
Flughafenstrasse 4
D-6000 Frankfurt-Niederrad(DE)

(72) Erfinder: Sonneborn, Hans, Dr.
Im Birkeneck 70
D-6056 Heusenstamm(DE)

(72) Erfinder: Schneider, Frank
Gartenstrasse 26
D-4700 Hamm 5(DE)

(72) Erfinder: Dörfelt, Bern
Stettiner Strasse 8/VI
D-6370 Oberursel 5(DE)

(72) Erfinder: Falk, Georg
Ahornweg 12
D-6072 Dreieich(DE)

(72) Erfinder: Kothe, Norbert, Dr.
Friedrich-Ebert-Strasse 21
D-6242 Kronberg/Ts.(DE)

(72) Erfinder: Apel, Kerstin
Pittlerstrasse 67
D-6070 Langen(DE)

(74) Vertreter: Beil, Walter, Dr. et al,
BEIL, WOLFF & BEIL Rechtsanwälte Adelonstrasse 58
D-6230 Frankfurt am Main 80(DE)

(54) Behälter für das Wachstum steriler Pflanzen.

(57) Die Erfindung betrifft einen Behälter für das Wachstum steriler Pflanzen, gekennzeichnet durch ein schalenartiges Gefäß (1) zur Aufnahme von Nähragar, eine über das Gefäß gestülpte und dasselbe keimdicht verschließende durchsichtige Glocke (2), einen unmittelbar über dem Gefäßrand sich befindlichen und um denselben herumlaufenden, zum Inneren das Gefäßes sich neigenden dachartigen Aufsatz (3) und eine im Inneren des Gefäßes in Höhe des vorgesehenen Agarspiegels sich befindliche Agarfixiereinrichtung (4).

Fig.

Unsere Nr. 23 886                    Pr/br

Biotest-Serum-Institut GmbH
Flughafenstraße 4
6000 Frankfurt 71

Behälter für das Wachstum steriler Pflanzen

Die Erfindung betrifft einen Behälter für das Wachstum steriler Pflanzen.

In jüngster Zeit bedient man sich zur Züchtung bzw. Vermehrung von Pflanzen sogenannter Gewebe-, Meristem- oder Zellkulturen. Dabei werden Pflanzenorgane oder Zellverbände von der sterilisierten oder aus sterilem Samen unter sterilen Bedingungen gezüchteten Stammpflanze isoliert, in ein steriles Nährmedium eingebracht und unter sterilen Bedingungen gezüchtet. Als Kulturgefäß verwendet man dabei beispielsweise Reagensgläser, Erlenmeyerkolben oder Marmeladengläser (vgl. "Zierpflanzenbau/Gartenbautechnik", Nr. 11, 27.5.1981). Derartige Gewebekulturen dienen jedoch lediglich Zuchtzwecken und nicht Zierzwecken. Wenn die auf diese Weise gezüchteten Pflanzen eine bestimmte Größe erreicht haben, werden sie in Erde verpflanzt und nach einer bestimmten Akklimatisierungszeit als normale Topfpflanzen in Erde unter nicht mehr sterilen Bedingungen weitergezüchtet bzw. in den Handel gebracht.

Aufgabe der Erfindung ist es, einen Behälter für das Wachstum steriler Pflanzen bereitzustellen, der sich für Zierzwecke eignet und der nicht nur ein ansprechendes Äußeres aufweist, sondern in nicht steriler Umgebung die Voraussetzungen für ein steriles Pflanzenwachstum erfüllt.

Diese Aufgabe konnte mit Hilfe des erfindungsgemäßen Behälters gelöst werden, der gekennzeichnet ist durch ein schalenartiges Gefäß (1) zur Aufnahme von Nähragar, eine über das Gefäß gestülpte und dasselbe keimdicht verschließende durchsichtige Glocke (2), einen unmittelbar über dem Gefäßrand sich befindlichen und um denselben herumlaufenden, zum Inneren des Gefäßes sich neigenden dachartigen Aufsatz (3) und eine im Inneren des Gefäßes in Höhe des vorgesehenen Agarspiegels sich befindliche Agarfixiereinrichtung (4).

Die wichtigsten funktionellen Kriterien für einen Behälter für das Wachstum steriler Pflanzen, wie er erfindungsgemäß Verwendung finden soll, sind absolute Dichtigkeit gegenüber Staub und Mikroorganismen, die Gewährleistung, daß kein Kondenswasserverlust auftritt und das Kondenswasser in den Agar zurückfließt und die Fixierbarkeit des Agars für Transportzwecke.

Darüberhinaus soll der Behälter leicht beschickbar und entleerbar sein, und unter dem Gesichtspunkt des Zierzweckes soll er so gestaltet sein, daß die Sicht auf die Pflanze nicht beeinträchtigt wird.

Die Dichtigkeit wird durch eine über das zur Aufnahme des Agars und der Pflanze bestimmte Gefäß gestülpte dichtschließende Glocke, die den Gefäßrand überlappt,erreicht, wobei als Verschluß beispielsweise ein Bajonettverschluß, ein Dichtungsring oder ein Schraubverschluß verwendet werden kann.

Um einen dichten Schluß zu erreichen, müssen die Konfiguration des Teils des Gefäßrandes, der durch die Glocke überlappt wird und der überlappende Teil der Glocke aufeinander abgestimmt sein. Dies wird beispielsweise dadurch erreicht, daß man ein Gefäß mit einem zylindrischen Außenumfang und eine zylindrische Glocke verwendet, wobei der äußere Umfang des Gehäuses sich bündig in den inneren Umfang der Glocke schiebt. Der äußere Umfang des Gefäßes und der Glocke müssen jedoch nicht zylindrisch sein. Sie können beispielsweise auch kegelstumpfförmig sein und können außer einer runden eine dreieckige, quadratische oder polygonale Grundfläche besitzen. Wichtig ist nur, daß Form von Gefäß und Glocke aufeinander abgestimmt sind.

Die Überlappung kann bis zum unteren Ende der äußeren Gefäßwand des schalenartigen Gefäßes reichen, sie kann jedoch auch an irgendeinem Punkt dieser Wand enden.

In einer bevorzugten Ausführungsform ist die äußere Gefäßwand an ihrem unteren Ende treppenförmig ausgebildet, und die Überlappung endet an dem außen liegenden Treppenabsatz. Dadurch erzielt man eine glatte Verbindungsstelle zwischen Gefäß und Glocke, wodurch sich die Gesamtkombination durch Umspritzen oder eine ähnliche umlaufende Verschlußart leicht versiegeln und ggf. mit einem Originalitätsverschluß versehen läßt.

Aus Gründen der Agarersparnis ist der Innenraum des Gefäßes vorzugsweise halbkugelförmig ausgebildet.

Der obere Abschluß der Glocke kann beispielsweise halbkugelförmig, zylindrisch oder spitz sein.

Um zu erreichen, daß das Kondenswasser in den Agar zurückfließt, erstreckt sich über den Umfang des Gefäßrandes eine Art Wasserableitungsnase, d.h. ein dachförmiger Aufsatz, dessen Dachschräge derart ist, daß das untere Ende des Daches über den Gefäßrand hinaus in das Gefäß hineinragt. Dadurch fließt das an den Wänden der Glocke sich sammelnde Kondenswasser in das Gefäß bzw. in den Agar zurück. Dieser Aufsatz kann fest mit der Wandung der Glocke verbunden sein. Er kann aber auch eine Einheit mit der Agarfixiereinrichtung bilden und sich beispielsweise zwischen Oberkante des Gefäßes und einer Aussparung in der Glockenwandung einschieben lassen.

Für Transportzwecke muß der vom Gefäß aufzunehmende Nähragar fixiert sein. Zu diesem Zweck befindet sich im Inneren des Gefäßes in Höhe des vorgesehenen Agarspiegels eine Agarfixiereinrichtung. Diese kann beispielsweise als flachliegendes Sieb ausgebildet sein, das in der Mitte ein Loch für die Pflanze aufweist. Dieses Sieb kann entweder an der Wandung des Gefäßes befestigt sein oder es kann mit dem Aufsatz eine Einheit bilden, wie bereits vorstehend im Zusammenhang mit dem Aufsatz beschrieben. Die Agarfixiereinrichtung kann auch die Form eines Wulstes haben, der sich um den Umfang der Innenwandung des Gefäßes erstreckt.

Die Kombination aus relativ flacher, leicht zugänglicher Schale und leicht abnehmbarer Glocke erlaubt eine leichte, problemlose Beschickung und Entleerung des Behälters im Gegensatz zu den bisher für Zuchtzwecke verwendeten Behälterformen, die hochrandig mit einem flachen Deckel sind und nur mit langen Pinzetten und ähnlichen Geräten zugänglich sind.

Außerdem erlaubt die durchsichtige Glocke eine ungehinderte Sicht auf die Pflanze.

Das Material der Glocke soll steriliserbar sein und kann beispielsweise aus Glas oder einem durchsichtigen Kunststoff bestehen.
Das Gefäßmaterial sollte ebenfalls sterilisierbar sein und aus Glas oder durchsichtigem Kunststoff bestehen, um die Sicht auf das Wurzelwachstum zu ermöglichen.

Die Abmessungen des Behälters unterliegen keinen bestimmten Grenzen und richten sich lediglich nach der Größe der zu züchtenden Pflanze. In einer zweckmäßigen Ausgestaltung kann die Grundfläche des Behälters 4 bis 6 cm, vorzugsweise 5 cm, und die Höhe der Glocke 10 bis 15 cm, vorzugsweise 11 cm, sein.

Beide Teile werden in sterilem Zustand in einer Sterilbank aufeinandergesetzt.

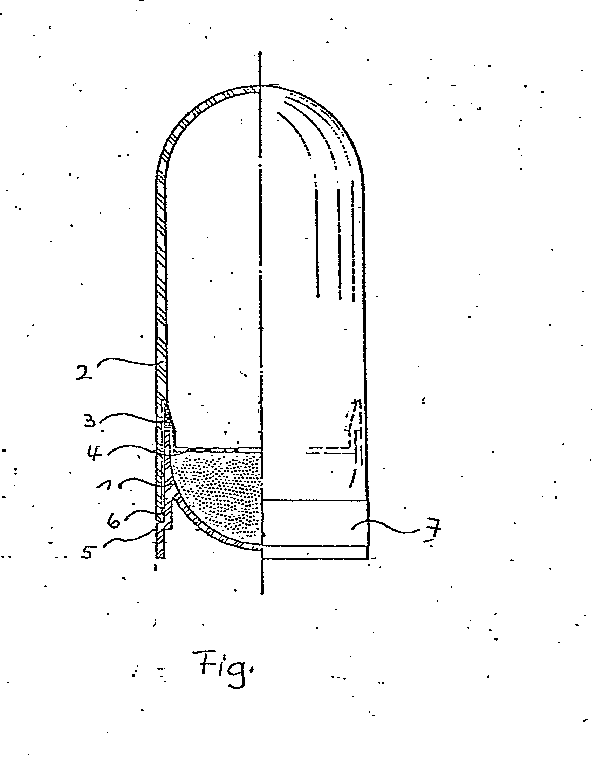

Die Erfindung wird im folgenden anhand der Zeichnung näher erläutert.

Die Figur stellt einen Aufriß mit halbseitigem Längsschnitt des erfindungsgemäßen Behälters dar. In der Figur ist das Gefäß (1) zu sehen mit einem zylindrischen äußeren Umfang und einem halbkugelförmigen Innenraum. Der äußere Umfang ist als Treppe (5) ausgebildet. Die Glocke (2) ist über das Gefäß (1) gestülpt. (3) ist der für die Kondenswasserableitung bestimmte Aufsatz, der in dieser Ausführungsform eine Einheit mit der siebartig ausgebildeten Agarfixiereinrichtung bildet. An der Stelle, an der der Aufsatz (3) beginnt, ist die Innenwand der Glocke (2) so verschmälert, daß der obere Teil des Aufsatzes (3) in den sich dabei bildenden Erker hineinpaßt und das überlappende Ende bündig mit der Treppe (5) im äußeren Umfang des Gefäßes abschließt. Am äußeren Umfang des Gefäßes (1) ist eine kleine Ausstülpung (6) eines Bajonettverschlusses zu sehen. Ferner wird ein umlaufender Verschluß (7) gezeigt, der ggf. einem zusätzlichen Versiegeln oder der Anbringung eines Originalitätsverschlusses dienen kann.

Patentansprüche

1. Behälter für das Wachstum steriler Pflanzen, gekennzeichnet durch ein schalenartiges Gefäß (1) zur Aufnahme von Nähragar, eine über das Gefäß gestülpte
und dasselbe keimdicht verschließende durchsichtige
Glocke (2), einen unmittelbar über dem Gefäßrand
sich befindlichen und um denselben herumlaufenden, zum
Inneren des Gefäßes sich neigenden dachartigen Aufsatz (3) und eine im Inneren des Gefäßes in Höhe
des vorgesehenen Agarspiegels sich befindliche
Agarfixiereinrichtung (4).

2. Behälter nach Anspruch 1, dadurch gekennzeichnet, daß
der Innenraum des Gefäßes (1) halbkugelförmig
ausgebildet ist.

3. Behälter nach Anspruch 1 oder 2, dadurch gekennzeichnet,
daß der äußere Umfang des Gefäßes (1) zylindrisch
ausgebildet und mit einer Stufe zur Aufnahme der
Glocke (2) ausgestattet ist.

Fig.

Europäisches
Patentamt

**EUROPÄISCHER RECHERCHENBERICHT**

**0079046**
Nummer der Anmeldung

EP  82 11 0148

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| A | US-A-4 224 765  (SONG)<br>* Spalte 2, Zeile 8 - Spalte 4, Zeile 21; Figuren 1-8 * | 1 | A 01 G  9/02<br>A 01 G  7/00 |
| A | FR-A-2 362 580  (AFIPLASTEX)<br>* Seite 3, Zeile 13 - Seite 5, Zeile 9; Figuren 2,3 * | 1,3 | |
| A | FR-A-2 122 819  (CONSO)<br>* Seite 2, Zeile 7 - Seite 3, Zeile 7; Figuren 1-5 * | 1,2 | |

RECHERCHIERTE
SACHGEBIETE (Int. Cl. ³)

A 01 G
C 12 M

Der vorliegende Recherchenbericht wurde für alle Patentansprüche erstellt.

| Recherchenort | Abschlußdatum der Recherche | Prüfer |
|---|---|---|
| DEN HAAG | 08-02-1983 | HERYGERS J.J. |